Europäisches Patentamt

European Patent Office

Office européen des brevets

(11) Publication number: **0 151 128 B1**

# EUROPEAN PATENT SPECIFICATION
## published in accordance with Art. 158(3) EPC

(45) Date of publication of patent specification: **03.07.91**

(51) Int. Cl.$^5$: **A61K 39/40**, G01N 33/569, C12P 21/00, C12N 15/00, C12N 5/00, C12N 1/00, C07C 7/00

(21) Application number: **84902150.6**

(22) Date of filing: **04.05.84**

(86) International application number: **PCT/US84/00688**

(87) International publication number: **WO 84/04458 (22.11.84 84/27)**

Divisional application 88105563 filed on 07.04.88.

The file contains technical information submitted after the application was filed and not included in this specification

(54) **MONOCLONAL ANTIBODIES REACTIVE WITH ENDOTOXIN CORE.**

(30) Priority: **06.05.83 US 492374**

(43) Date of publication of application:
**14.08.85 Bulletin 85/33**

(45) Publication of the grant of the patent:
**03.07.91 Bulletin 91/27**

(84) Designated Contracting States:
**AT BE CH DE FR GB LI LU NL SE**

(56) References cited:
**US-A- 4 196 265**
**US-A- 4 237 224**

(73) Proprietor: **VELOS GROUP**
**4824 Montgomery Lane**
**Bethesda, Maryland(US)**

(72) Inventor: **POLLACK, Matthew**
**5817 Wyngate Drive**
**Bethesda MD 20817(US)**
Inventor: **HUNTER, Kenneth W.**
**4401 Dresden Street**
**Kensington MD 20895(US)**

(74) Representative: **Patentanwälte Grünecker, Kinkeldey, Stockmair & Partner**
**Maximilianstrasse 58**
**W-8000 München 22(DE)**

PROCEEDINGS OF THE NATIONAL ACADEMY OF SCIENCES USA, vol. 79, March 1982, pages 1616-1620; J. HIERNAUX et al.: "Shared idiotypes among monoclonal antibodies specific for different immunodominant sugars of lipopolysaccharide of different Gram-negative bacteria"

BIOLOGICAL ABSTRACTS, vol. 66, August 1, 1978, abstract 14686; M. TYOKU et al.: "Easily inducible M-component-like IgG3 antibody to LPS in mice, & J. EXP. MED. 46(6): 415-417

BIOLOGICAL ABSTRACTS, vol. 76, 1983, abstract 65017; J.R. HIERNAUX et al.: "Study of the idiotypy of lipopolysaccharide-specific polyclonal and monoclonal antibodies", & EUR. J. IMMUNOL. 12(10): 797-803

Nature, vol. 256, Aug. 1975, (Eng), Köhler, G., and Milstein, C., "Continuos cultures of fused cells secreting antibody of predefined specificity". see pp 495-497

Infection and Immunity, vol. 40, no. 2, May 1983 (Eng), Huber-Lukac et al., "Specificities of Monoclonal antibodies against the activated Endotoxin of Bacillus thuringiensis var. thuringiensis", see pp 608-612.

Infection and Immunity, vol. 42, no. 3, Dec. 1983 (Eng), Sugasawara, R. et al.: "Monoclonal Antibodies against Neisseria meningitidis lipopolysaccharide", see pp. 863-868

Infection and Immunity, vol. 38, no. 2, Nov. 1982 (Eng.) Gustafsson, B. et al.: "Monoclonal antibodies against vibrio cholerae lipopolysaccharide" see pp 449-454

Chemical Abstracts, vol. 97, no.19, issued 1982 (Columbus, Ohio, US), Ono E. et al., "Production and characterization of monoclonal antibodies to lipopolysaccharide antigen of Leptospira interrogans Serovar Kremastor and canicola". See p 523, col. 2, Abstract 160756g, Zentralbl. Bakteriol. Mikrobiol. Hyg. Abt. 1, Orig. A 1982, 252(3), 414-24 (Eng)

Young "Clinical Research", vol. 30, 02.04.89

Young's declaration and exibits 1 to 6

Infection and Immunity, vol. 34, no. 3, issued Dec. 1981 (Eng) Apicella, M. et al.: Monoclonal Antibody Analysis of lipopolysaccharide from Neisseria gonorrhoeae& Neisseria meiningitidis", see pp 751-756

Clinical Research, vol. 30, no.2, April 1982, abstract 522A:"Monoclonal antibody directed against the "core" glycolipid of enterobacterial endotoxin"

Clinical Research, vol. 30, no. 2, 1982, abstract 376A:"Enhanced survival following pseudomonas aeruginosa (PA) septisemia in patients with high serum antibody titers to escherichia coli core glycolipid (CGL)"

J.CLIN.INVEST., vol. 69, April 1982, pp. 742-749

CLINICAL RESARCH, vol. 38, no.2, April 1990, ref.: 304A:"Prevention of death from gram negative bacteremia and sepsis..."

PROC.NATL.ACAD.SCI. USA, vol. 82, March 1985, pp. 1790-1794

PROG.CLIN.BIOL.RES., vol.272, 1988, pp. 383-393

SURGERY, vol. 106, 1989, pp. 147-155

SURGERY, vol. 98, 1985, pp. 283-288

ABSTRACTS OF THE TWENTY-NINTH INTER-SCIENCE CONFERENCE ON ANTIMICROBIAL AGENTS AND CHEMOTHERAPY, 17-20 Sept. 1989, Houston, Texas, abstract no. 1101

ARCH.SURG., vol. 121, Jan 1986, pp. 58-61

Infectious diseases vol 159 no. 2 Feb. 1989 pp 168-188

Infectious diseases vol. 159 no. 5 May 1989 pp 872-880

Infection and Immunity vol. 55 Nov. 1987 pp 2636-2644

Medical micro-biology vol 26 1988 pp. 107-114

Infection & Immunity vol. 52 no 1 April 1986 pp 56-62

EP 0 151 128 B1

## Description

### U.S. GOVERNMENT RIGHTS

The invention described herein may be manufactured, used and licensed by or for the U.S. Government for governmental purposes only without the payment to the inventors of any royalties thereon.

### BACKGROUND OF THE INVENTION

This invention is related to the production and use of novel monoclonal antibodies reactive with at least part of the endotoxin core of Gram-negative bacteria, and self-reproducing carrier cells containing genes which code for monoclonal antibodies reactive with endotoxin core. The invention is directed to the antibodies, to processes of preparing the antibodies, to use of the antibodies in diagnostic, prophylactic, and therapeutic methods and to compositions employing the antibodies, and to investigational, pharmaceutical, and other methods and compositions employing the antibodies.

Gram-negative bacteria are a ubiquitous and diverse group of microorganisms that cause a number of serious and often life-threatening infections. Lipopolysaccharides are the principal biochemical constitutents of the external covering or cell wall which characterizes all Gram-negative bacteria. These lipopolysaccharides, or endotoxins as they are called, play a major pathophysiologic and immunologic role in Gram-negative infections. Antibodies directed toward bacterial lipopolysaccharides constitute a critical host defense against these toxic substances and against the organisms which produce them.

In addition to their direct role in Gram-negative infections, endotoxins possess diverse and potent biological activities which, when coupled with the widespread presence of endotoxins in man's internal and external environments, endow them with great medical, scientific, and economic importance.

In general, bacterial lipopolysaccharides consist of a highly variable outer region composed of repeating oligosaccharide (sugar) units comprising the so-called "O-specific side-chain," and a relatively constant core region containing a limited number of sugars, often including the trisaccharide 2-keto-3-deoxyoctonate (KDO), and the biologically active lipid A moiety. Lipid A's derived from a number of distinct bacterial groups show a close structural relationship, and in most cases studied, lipid A consists of a $\beta$1,6-linked glucosamine disaccharide with ester- and/or amide-linked long chain fatty acids and with other possible substitutions. The core region, which is sometimes referred to as core glycolipid or endotoxin core, may be considered a lipopolysaccharide (or endotoxin) of Gram-negative bacteria that is lacking its O-specific side-chain. In addition, the endotoxin core structure may itself be incomplete on the basis of missing core sugars and/or other substituents. Thus, endotoxin core is incomplete lipopolysaccharide, lacking part or all of the O-specific side-chain, and, in some cases, also lacking core sugars and/or other substituents while usually retaining lipid A. (See E. Th. Rietschel et al., Scandinavian Journal of Infectious Diseases, Supplement 31:8-21, 1982.

Significantly, most Gram-negative bacteria, representing diverse genera and species and including almost all which are pathogenic for man, share highly analogous, immunologically cross-reactive endotoxin core structures. For purposes of this specification, endotoxin core will be defined as that part of the lipopolysaccharide or endotoxin of Gram-negative bacteria comprised of complete or incomplete, substituted or unsubstituted lipid A covalently bound to substituted and/or unsubstituted core sugars, said lipid A characterized, for example, by a phosphorylated $\beta$1,6-linked glucosamine disaccharide with ester- and/or amide-linked long chain fatty acids, and said core sugars distinguished by their location in an interconnecting position between lipid A and the repeating oligosaccharide units of the O-specific side chain in the intact lipopolysaccharide molecule.

Antibodies to O-specific side chains in the variable outer region of lipopolysaccharides are species- and type- specific, with protective activity derived from their ability to promote the phagocytosis (engulfment) and killing of infecting organisms by host phagocytes (white blood cells). In contrast, antibodies directed toward the lipid A-containing inner core region are broadly cross-reactive among a wide variety of Gram-negative bacteria and are thought to act by neutralizing the biological activities of endotoxin.

Antibodies are produced by living cells called plasma cells which are specialized for that function. Plasma cells are derived from other cells called B lymphocytes which bear receptors on their cell membrane with the same antigen specificity as the antibodies synthesized and secreted by the plasma cells. Each immunocompetent B lymphocyte and its progeny (clone) bear receptors with unique specificity. Foreign substances (antigens) bind to receptors on B lymphocyte cell membranes and stimulate these specific B lymphocyte clones to proliferate, differentiate into plasma cells, and produce specific antibodies. In simplified terms, there are as many lymphocyte clones as there are specific antibodies, and as many

4

specific antibodies as there are distinct antigens. Conversely, each specific antibody is produced by plasma cells derived from a single clone of immuno-competent lymphocytes, and an individual exposed to (i.e., immunized with) a specific antigen, produces specific antibody to that antigen through expansion of the appropriate lymphocyte clone.

Antibodies are characterized by exquisite specificity for the antigen toward which they are directed. In reality, most antigens contain more than one antigenic site, so that multiple antibodies may be directed at a single antigens. In addition, different antibodies with varying affinities (strength of antigen binding) may be directed toward single antigenic sites. Since multiple antibodies directed toward the same antigen are derived from different lymphocyte clones, they are referred to as "polyclonal" antibodies. The normal antibody response to most antigens is polyclonal. At the same time, a single antibody may react with multiple antigens or antigenic sites which have common or analogous molecular structures; such an antibody is called a cross-reacting antibody.

It can thus be appreciated that the total antibody repertoire of an individual is enormous in respect to both size and breadth, and that natural exposure of an individual to an invading microorganism or immunization with a complex antigen will result in a polyclonal antibody response. The serum from such an individual will contain a complex admixture of pre-existing and new antibodies characterized by a multitude of specificities and affinities.

Traditionally, polyclonal antibodies have been prepared by immunizing an animal or man with the material (antigen) toward which antibodies are sought. If the goal of immunization is the prevention or treatment of a specific disease, intoxication or infection, an individual may be immunized directly with the appropriate antigen (active immunization), or administered preformed antibodies or immune serum prepared by prior immunization of another individual with the same antigen (passive immunization). Both types of immunization have major shortcomings. In the case of active immunization, there may be insufficient time to achieve an adequate antibody response to prevent or treat a particular infection or disease. In addition, it is sometimes impossible to accomplish active immunization because of ineffective vaccines, the inability of certain groups (e.g., immuno-suppressed persons and infants) to respond to vaccination, or untoward reactions sometimes associated with active immunization. Passive immunization, on the other hand, lacks specificity and is associated with a significant risk of transmissible infections such as hepatitis or other adverse reactions. Antisera, or immunoglobulins prepared from antisera, contain not only the desired antibody, but literally thousands of other antibodies as well. In fact, the desired antibody usually represents only a small fraction of the total antibody present in such antisera. It may be difficult, therefore, to achieve adequate levels of this antibody through passive immunization using such antisera. This poses additional risks to the patient as the infusion of large volumes of antisera or immunoglobulin greatly increases the likelihood of serious adverse reactions and infusion-related infections.

The non-therapeutic uses of polyclonal antibodies, as for example in immuno-logical research and various biotechnological applications, may also be seriously hampered by the variability of antibody responses to many antigens and the lack of specificity of antisera which contain a wide variety of antibodies.

Thus, the heterogeneity and diversity of naturally acquired or immunization-induced antibodies, and the unpredictability of antibody responses to antigenic stimuli, are factors which seriously limit the practical use of these polyclonal antibodies for clinical or scientific purposes.

While the foregoing discussion of the limitations of polyclonal antibodies has been stated in general terms, these same limitations apply to the therapeutic and non-therapeutic uses of polyclonal antibodies directed toward endotoxin core specifically.

In summary, Gram-negative bacteria are a widely prevalent group of microorganisms that commonly cause serious and often life-threatening infections. These bacteria all produce lipopolysaccharides or endotoxins which confer upon them important pathogenic and immunologic properties. Endotoxins have broad medical, sciencific and economic significance on the basis of their varied biological properties that goes well beyond their role in Gram-negative infections. Endotoxins from a wide variety of sources share a common or highly analogous core structure. Antibodies to this core structure cross-react with lipopolysaccharides produced by many different Gram-negative bacteria. These cross-reactive antibodies neutralize the biological activities of endotoxin, and appear to provide protection against serious Gram-negative infections. The utilization of naturally acquired or immunization-induced polyclonal antibodies reactive with endotoxin core is limited by the low immunogenicity of endotoxin, the lack of specificity of polyclonal antiserum, and the adverse reactions associated with conventional active or passive immunization.

SUMMARY OF THE INVENTION

5

In contrast with multiple or polyclonal antibodies are single or "monoclonal" antibodies. These are antibodies derived from a single lymphocyte clone, which makes them absolutely specific and homogeneous. In 1975, Kohler and Milstein (Nature, 265:495, 1975), first described how monoclonal antibodies directed to sheep red blood cells may be prepared by fusing a specific antibody-producing B lymphocyte with a tumor cell, resulting in an "immortal" self-reproducing hybrid clone (or "hybridoma") that can synthesize, in a test tube (in vitro) or an animal (in vivo), a single, monoclonal antibody. Such a hybridoma is, in effect, a self-reproducing cell "factory" which can produce a potentially limitless supply of an antibody with single, pre-defined specificity.

Monoclonal antibodies having a single, predefined specificity are already known. Apicella et al. disclose a hybridoma which was produced by fusion of plasmacytoma cells to splenocytes harvested from mice immunised with whole gonococci (Infection and Immunity 34, pages 751 to 756, 1981). Antibodies secreted by these hybridomas react with the carbohydrate portion of the gonococcal LPS. Further, a method for the production of these hybridomas is disclosed. A process for the production of monoclonal antibodies directed to gonococcal LPS is also disclosed by Morse and Apicella (Journal of Infectious Diseases 145, pages 206 to 216, 1982). According to their immunization protocol also whole microorganisms were used as antigenic material.

WO-A- 85/01659, which was published later than the filing date of the present application, refers to several documents concerning antibodies against antigenic regions in LPS. For example Luderitz (Luderitz et al., Curr.Top.Membr.Transp.17, pages 79 to 151, 1982) discloses, that there are at least 3 different antigenic regions in endotoxins; Ziegler et al. (J. Immunology 111, pages 433 to 439, 1973) discloses the preparation of a vaccine from E.coli J5, which confers protection against lethal bacteremia caused by different Gram-negative bacteria in immuno suppressed animals. McCabe and colleagues showed that rabbit antisera to rough mutants of Salmonella minnesota protected granulocytopenic rabbits against lethal bacteremia and protected mice against lethal challenge with heterologous endotoxins. Ziegler et al. showed (New Eng.J.Med. 307, pages 1225 to 1230, 1982) the preparation of antisera reacting with the anticore glycolipid of the LPS, by vaccinating human subjects. Upon administration to gravely ill bacteremic patients the death rate from bacteremia decreased considerably.

We undertook to prepare novel self-reproducing cell lines which synthesized monoclonal antibodies directed toward endotoxin core, since such antibodies, if indeed, they could be produced, would satisfy a number of critical needs not fulfilled by existing polyclonal or monoclonal antibody technology. Like polyclonal antibodies to endotoxin core, such monoclonal antibodies would be broadly cross-reactive among a wide variety of pathogenic Gram-negative microorganisms, and would therefore have great potential utility in the prevention and treatment of diseases due to these bacteria. However, they would be more useful than polyclonal antibodies as immunoprophylactic, therapeutic, and diagnostic reagents because of their exquisite specificity. Likewise, this specificity would also render such monoclonal antibodies more useful for immuno-logical and biochemical studies of endotoxin, for affinity purification of endotoxin, and for the neutralization and/or removal of endotoxin from pharmaceuticals and other reagents. Furthermore, unlike conventional polyclonal antibodies, monoclonal antibodies reactive with endotoxin core could be produced in a potentially limitless and homogeneous supply, thus avoiding the problems imposed by the low immunogenicity of endotoxin, the variability of polyclonal antibody responses, and the resulting limitations in achievable levels or supplies of anti-endotoxin antibodies available for clinical and other applications.

It is one object of the present invention to provide self-reproducing carrier cells having deposit number ATCC HB 8297 and ATCC HB 8298, containing genes that code for the production of monoclonal antibodies reactive with endotoxin core.

It is a further object to provide the antibodies so produced.

A still further object is to provide an in vitro process for producing these antibodies.

Still another object is to provide research compositions containing the antibodies useful for immunological or biochemical anlalyses of endotoxins.

An even further object is to provide compositions containing the antibodies suitable for isolating or purifying endotoxin from mixtures containing endotoxin and other substances.

Another object is to provide compositions containing the antibodies useful for the neutralization and/or removal of endotoxin from other material or solutions.

Another object is to provide compositions for using antibodies reactive with at least part of the endotoxin core of Gram-negative bacteria in the diagnosis, prophylaxis and treatment of disease caused by endotoxin bearing pathogens.

Other objects and advantages of the present invention will be set forth in part in the description which follows, and in part will be obvious from the description, or may be learned by practice of the invention.

In satisfaction of the foregoing objects and objectives there is provided by this invention monoclonal

6

antibodies reactive with endotoxin core. The antibodies are produced by self-reproducing carrier cells containing genes that code for monoclonal antibodies reactive with endotoxin core.

Additionally, there are provided in satisfaction of the foregoing objects and objectives in vitro processes that include culturing the above mentioned carrier cells in a suitable medium for an appropriate period of time, and recovering the antibodies from the medium in which the carrier cells are grown.

Also provided by this invention is a method for the immunological detection of endotoxin, or for the diagnosis, in a human or an animal, of an infection caused by pathogenic microorganisms bearing endotoxin. This method includes mixing a diagnostically effective amount of the antibodies of this invention with a sample of endotoxin-containing solution such as a body fluid or tissue removed from the man or animal and measuring the degree of the reaction in the resulting mixture. The present invention also provides compositions for the detection of endotoxin or for the diagnosis of an infection caused by an endotoxin-bearing microorganism. These compositions include, in admixture with a diagnostically acceptable carrier, a concentration of the antibodies according to the present invention effective to detect endotoxin or to diagnose the infection.

Also provided are compositions for passive prophylaxis or therapy of such an infection, the compositions including, in admixture with a physiologically acceptable carrier, a concentration of antibodies reactive with at least part of the endotoxin core of Gram-negative bacteria effective to result in passive prophylaxis or treatment.

There is also provided by this invention research compositions useful for carrying out immuno-logical and biochemical analyses of endotoxins. These compositions include, in admixture with a carrier suitable for research, an amount of the antibody effective to provide such information when it is mixed with endotoxins.

There are further provided by this invention compositions useful for the isolation and purification of endotoxins from mixtures or solutions which contain them, the compositions including a suitable matrix for support of the antibodies effective in permitting isolation and purification of endotoxins by immune absorption.

There is still further provided by this invention compositions useful for the neutralization and/or removal of endotoxins from materials or solutions which contain them, such compositions including a suitable carrier or matrix to permit precipitation or immune absorption of these endotoxins from such materials or solutions which contain them.

The accompanying drawings and tables, which are incorporated in and constitute a part of this specification, illustrate and together with the description serve to explain the principle of the invention.

## BRIEF DESCRIPTION OF THE DRAWINGS

Figure 1 depicts an analysis of purified endotoxin core by electrophoresis on 14% SDS polyacrylamide gel.

Figure 2 depicts the binding in an ELISA assay of monoclonal antibodies obtained from a J5-1 hybridoma culture supernatant with purified Escherichia coli J5 endotoxin core.

Figure 3 shows the binding in an ELISA assay of monoclonal antibodies produced by a J5-1 ascites tumor with purified Escherichia coli J5 endotoxin core.

Figure 4 is a depiction of the results of a competitive inhibition ELISA assay illustrating a concentration-dependent inhibition of the specific binding activities of the J5-1 and J5-2 monoclonal antibodies by homologous and heterologous endotoxins.

## DESCRIPTION OF THE PREFERRED EMBODIMENTS

The preparation and characterization of self-reproducing carrier cells ATCC HB 8297 and ATCC HB 8298 and resulting antibodies reactive with endotoxin core as well as various methods and compositions employing the antibodies, will be better understood by reference to the following description, which sets forth the preferred embodiments of the invention.

The carrier cell lines embraced by this invention are hybridomas ATCC HB 8297 and ATCC HB 8298. Biologically pure cultures are available from the permanent collection of the American Type Culture Collection, 12301 Parklawn Drive, Rockville, Maryland, U.S.A. 20852 and were deposited on May 6, 1983.

As indicated, the scope of the present invention embraces self-reproducing carrier cells having the characteristics of ATCC HB 8297 and ATCC HB 8298 containing genes that code for the production of monoclonal antibodies reactive with any part of endotoxin core of Gram-negative bacteria. This specification describes in detail the steps taken by the inventors to produce the above ATCC HB 8297 and ATCC HB 8298.

7

To make hybridomas (fused cells) that secrete monoclonal antibodies reactive with endotoxin core, in accordance with the invention, the following procedures were used.

The double, rough mutant derived from an E. coli 0111:B4 parent strain and designated "J5" (ATCC 39355, available from the American Type Culture Collection, 12301 Parklawn Drive, Rockville, Maryland, U.S.A. 20852 and deposited on May 6, 1983) lacks the enzyme UDP-galactose 4-epimerase and is unable to incorporate exogenous galactose into its lipopolysaccharide structure. As a consequence of this mutation, the endotoxin produced by this strain lacks O-specific side chains and outer core sugars, and consists only of lipid A and sugars present in the inner core (in this case comprising 2-keto-3-deoxyoctonate, heptose, glucose, and N-acetylglucosamine). The J5 strain was used in this case by way of example only. For the purposes of the invention, any bacterial strains bearing an endotoxin similar to that of the J5 strain could be used as a source of endotoxin core. So-called "rough" mutants are the preferred organisms for this purpose since their endotoxin, like that of the J5 strain, lacks O-specific side chains and at least part of the outer core structure, thus exposing immunodeterminants associated with the cross-reacting inner core. Alternatively, endotoxin might be obtained from so-called "smooth" organisms whose lipopolysaccharide structure is relatively complete. The endotoxin from such strains could be physically or chemically degraded to yield a core structure analogous to that obtained from rough mutant strains, such as the J5 mutant, useful in the production of monoclonal antibodies reactive with endotoxin core.

The bacteria described above were grown overnight at $32^\circ$ C on tryptic soy agar slants (Difco, Detroit, MI) and then innoculated into 4 liter-capacity Fernbach flasks containing 1000 ml of tryptic soy broth (Difco). These cultures were incubated, with shaking, at $32^\circ$ C for 18 hr, the cells removed by centrifugation, washed twice with 0.15 M NaCl and weighed. The bacteria were then suspended at a concentration of 0.2 g/ml in 0.05 M Tris (hydroxymethyl)aminomethane-hydrochloride buffer, pH 9.0, with 0.15 M NaCl, sonicated with a large probe (Sonicator Cell Disruptor, Heat Systems-Ultrasonics, Inc., Plainview, NY) at maximum setting for a total of 10 min. After centrifugation the pellet was resuspended and the sonication repeated. The fragmented cells were suspended at a concentration of 0.4 -g/ml in distilled water and heated at $70^\circ$ C in a water bath; then an equal volume of 90% re-crystalized phenol (pH 7.0) was added, and the mixture incubated at $70^\circ$ C with constant stirring for 15 min (Westphal et al. Z. Naturforsch, 7b:148, 1952,). The mixture was then cooled to $4^\circ$ C, centrifuged at 10,000 rpm for 10 min, the aqueous phase saved, and the phenol phase reextracted with an equal volume of water as described above. The phenol phase and denatured protein at the phenol-water interface were discarded and the lipopolysaccharide-containing aqueous phases combined. The lipopolysaccharide suspension was then placed in a separatory funnel, extracted with anhydrous diethyl ether (2:1 ratio of ether to lipopolysaccharide suspension), and the aqueous phase saved. Water was added to the ether phase in the amount removed, the mixture shaken, and the aqueous phase again removed. The combined aqueous phases were re-extracted overnight with additional ether. Excess ether was removed from the final aqueous phase by bubbling nitrogen through the solution. RNAse (Type II-A, Sigma Chemical Co., St. Louis, MO) was added to the lipopolysaccharide suspension at a concentration of 2 Kunitz units/ml. The mixture was then dialyzed at room temperature against multiple changes of 0.01 M Tris-acetate, pH 7.5, with 0,1 M NaCl and 1.0 mM $NaN_3$ until the optical density of the dialysate, monitored in the 230-300 nm range reached 0. The lipopolysaccharide suspension was next dialyzed against water in the cold overnight. DNAse (Type II, Sigma) was added at a concentration of 2 Kunitz units/ml, and the suspension dialyzed against repeated changes of 0.1 M NaCl with 5 mM $MgSO_4$ and 1 mM $NaN_3$ until the absorbance of the dialysate reached 0 in the 230-300 nm range. The lipopolysaccharide suspension was then dialyzed against cold water overnight, pronase (Type V, Sigma) added in a concentration of 1 Kunitz unit/100 ml, the solution dialyzed overnight against several changes of 0.01 M Trisacetate pH 7.5, followed by dialysis against multiple changes of cold water over several days. The lipopolysaccharide suspension was lyophilized and weighed.

Whereas in this case endotoxin core was prepared as described above, alternative purification procedures, such as that described by Galanos et al. (European Journal of Biochemistry, 9:245, 1969), which is hereby incorporated by reference, might also be employed for the isolation of endotoxin core. In certain circumstances it might even be advisable to employ other purification methods, such as that of Galanos et al., in order to maximize the yield of endotoxin core or to retain or more favorably "present" particular antigenic sites or epitopes toward which monoclonal antibodies are sought.

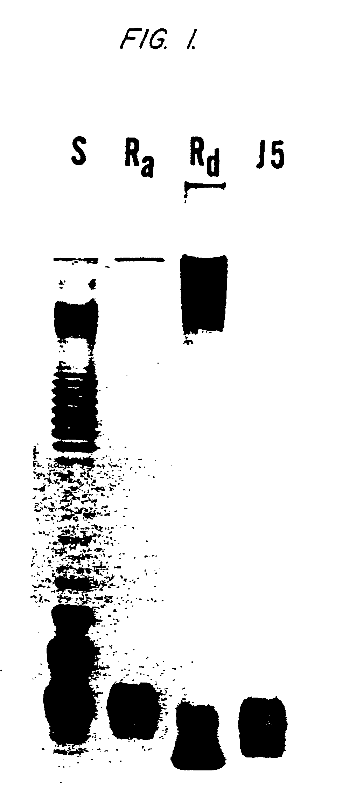

Purified E. coli J5 endotoxin core contained <1% protein as determined by the Coomassie Blue-binding method of Bradford (Bradford, M.M., Analytical Biochemistry, 72:248, 1976) and corroborated by amino acid analysis. Neutral sugar analysis by reverse-phase chromatography using a cation exchange resin in the $Li^+$ form with 90% ethanol as eluent revealed the presence of heptose and glucose, ± galactose, and no colitose, the latter being a unique sugar contained in the O-specific side chains of the smooth E. coli 0111:B4 parent strain. These data confirmed an incomplete core structure corresponding to the Rc chemotype of Salmonella (Luderitz, O., et al. Bacterological Reviews, 30:192, 1966; Wilkinson, S.G., In Surface Carbohydrates of the Prokaryotic Cell, I.W. Sutherland, editor, Academic Press, New York, 1977, pp 97-171). Electrophoresis in 14% SDS polyacrylamide gel, followed by silver staining (Figure 1), revealed a single, broad, fast-migrating band (J5) similar in mobility to core structures from other simultaneously run lipopolysaccharides (S, Ra, Rd); regularly spaced slower migrating bands characteristic of intact smooth lipopolysaccharide (S) were absent (see Tsai, C., Frasch, C.E., Analytical Biochemistry, 119:115, 1982). As depicted in Figure 1, the sample designated "S" contained 5 $\mu$g of lipopolysaccharide from the smooth Escherichia coli 0111:B4 wild type strain; "$R_a$" contained 0.2 $\mu$g of lipopolysaccharide from the E. coli PL2 rough mutant, characterized by a complete core structure corresponding to the $R_a$ chemotype of Salmonella; "$R_d$" contained 0.2 $\mu$g of lipopolysaccharide from the E. coli PL2-CL29 rough mutant, characterized by an incomplete core structure corresponding to the $R_d$ chemotype of Salmonella; and "J5" contained 0.2 $\mu$g of lipopolysaccharide from the uridine diphosphate 4-epimerase-deficient mutant designated J5, which is derived from the E. coli 0111:B4 parent strain, the incomplete core structure of which corresponds to the $R_c$ chemotype of Salmonella. The purity and functional integrity of endotoxin core prepared from J5 were confirmed in mitogenesis assays employing spleen cells from high- and low-responder C3H/FeJ and C3H/HeJ mice, respectively. Mitogenic responses to purified endotoxin core were comparable to those induced by highly purified lipopolysaccharide from the smooth E. coli K235 strain (Skidmore, B.J., Journal of Experimental Medicine, 142:1488, 1975). These included high responses by C3H/FeJ spleen cells, low or absent responses by C3H/HeJ spleen cells, and complete abrogation of mitogenic activity following incubation of endotoxin core with polymyxin B.

Antibodies to purified endotoxin core were quantified using an enzyme-linked immunosorbent assay (ELISA). Purified endotoxin core was dissolved at a concentration of 25 $\mu$g/ml in coating buffer (15 mM $Na_2CO_3$, 30 mM $NaHCO_3$, 3 mM $NaN_3$, pH 9.55) and dispensed in 50 ul aliquots into 96-well polystyrene microtiter plates (Dynatech Laboratories, Inc., Alexandria, VA). After overnight incubation at 4°C, the endotoxin suspension was removed and the wells washed five times with PBS-Tween (150 mM NaCl, 6 mM $Na_2 HPO_4$, 1 mM $KH_2PO_4$, 3 mm $NaN_3$, and 0.5 ml/l Tween-20). 50 ul test samples were added to wells, the plates incubated at 4°C for 30 min and then washed five times with PBS-Tween. The final three steps, separated by PBS-Tween washes, were as follows: addition of 50 ul of rabbit anti-mouse kappa-chain (Cappel Laboratories, Cochransville, PA) diluted 1:500 and previously absorbed with whole E. coli J5 cells, followed by incubation at 4°C for 30 min; addition of 50 ul of goat anti-rabbit IgG-alkaline phosphatase conjugate (Sigma) diluted 1:250 and incubation of 4°C for 30 min; addition of 50 ul of p-nitro phenyl-phosphate (Sigma-104) substrate, 1 mg/ml in 10% diethanolamine, pH 9.8 and incubation at 25°C for 60 min. Absorbance was read at 405 nm in a Titertek Multiskan micro-ELISA spectrophotometer (Flow Laboratories, Vienna, VA).

The assay was later standardized with affinity-purified endotoxin core-specific immunoglobulin prepared from high-titered mouse ascites fluid. Test sample dilutions were used which yielded an absorbance closest to the mid-point of the standard curve, and concentrations of specific antibody calculated by least squares method from the standard curve. The sensitivity of the assay was 0.02 $\mu$g/ml and reproducibility among triplicate samples averaged ± 4%.

The following is a description of how to prepared self-reproducing carrier cells, which in this case were mouse hybridomas, that contain genes coding for the production of monoclonal antibodies reactive with endotoxin core, and how clones of these carrier cells might be made to reproduce on a large scale, and how monoclonal antibodies might be obtained from these clones.

We describe how spleen cells (B lymphocytes) obtained from mice previously immunized with J5 endotoxin core were fused with mouse plasmacytoma cells, resulting in so-called hybridomas, and how cells from these hybridomas which secreted monoclonal antibodies to endotoxin core were cloned. We also demonstrate how two distinct hybridoma clones (J5-1 and J5-2), derived from two separate fusion experiments, were reproduced on a large scale employing both in vitro and in vivo techniques, and how monoclonal antibodies reactive with endotoxin core were obtained from these self-reproducing carrier cells in large, usable quantities.

Six week-old female BALB/c mice were injected intraperitoneally at three weekly intervals with 50 $\mu$g of purified J5 endotoxin core suspended in 0.15 M NaCl. The donor mice were killed by cervical dislocation three days following the last immunization; the spleen was removed aseptically and placed in a 35 mm plastic Petri dish with 5 ml of cold RPMI 1640 medium. The spleen was dissociated into a single cell suspension, the cells were washed twice with cold RPMI 1640, and resuspended in the same medium.

An immunoglobulin non-secreting mouse plasmacytoma cell line (P3-X63-Ag8.653) deficient in the enzyme hypoxanthine guanine phophoribosyl transferase (HGPRT-, EC 2.4.2.8), as disclosed by Kearney (Journal of Immunology, 123: 1548, 1979), was used as the fusion partner. This cell line is available from the American Type Culture Collection, Rockville, Maryland, where it is designated ATCC CRL-1580. The plasmacytoma cell line was maintained in RPMI 1640 medium containing 10% fetal bovine serum and further supplemented with 2 mM L-glutamine, 1% sodium pyruvate, 1% non-essential amino acids, 100 IU/ml penicillin and 100 $\mu$g/ml streptomycin. For three days prior to fusion, 0.1 mM 8-azaguanine was added to the plasmacytoma cells in order to kill any HGPRT+ revertants. On the day of fusion, the plasmacytoma cells were harvested from 75 cm$^2$ culture flasks, washed once and resuspended in serum-free RPMI 1640 medium. The plasmacytoma and previously harvested spleen cells were counted and their viability assessed by Trypan blue dye exclusion.

The fusion technique was modified from that of Gefter et al. (Somatic Cell Genetics, 3:231, 1977). Described below is the fusion experiment which yielded the J5-1 hybridoma clone; a second fusion experiment performed in in identical manner produced the J5-2 hybridoma clone. To a sterile 50 ml conical plastic tube was added 1.0 x 10$^8$ spleen cells and 1.0 x 10$^7$ plasmacytoma cells. The plasmacytoma-spleen cell suspension was centrifuged at 250 x g for 10 min at room temperature and the medium then decanted to near dryness. The cell pellet was loosened gently by flicking and 1 ml of 50% polyethylene glycol (MW 1400) in RPMI 1640, without serum, was added in drops over 45 seconds. The tube was gently agitated during this process. One minute later, another 2 ml of RPMI 1640 was added over 2 min. An additional 20 ml of RPMI 1640 was added over the next 2 min, and the cells pelleted by centrifugation at 250 x g for 10 min at room temperature.

The fluid was decanted and 40 ml of enriched selection medium was added. This medium was composed of Dulbecco's MEM containing 10% fetal bovine serum and supplemented with 2 mM L-glutamine, 1% sodium pyruvate, 1% non-essential amino acids, 100 IU/ml penicillin, 100 $\mu$g/ml streptomycin, 1 mM oxaloacetic acid, 10% NCTC 109, and 0.2 $\mu$g/ml bovine pancreatic insulin. The medium also contained 1.0 x 10$^{-4}$ M hypoxanthine, 4.0 x 10$^{-7}$ M aminopterin, and 1.6 x 10$^{-5}$ M thymidine (HAT). Aminopterin is toxic for cells that lack the enzyme HGPRT and therefore kills all unfused plasmacytoma cells. Fused cells (hybridomas) survive in HAT because they obtain HGPRT from the B lymphocyte (spleen cell) fusion partner.

Aliquots of 0.2 ml containing 5.0 x 10$^5$ cells were transferred from the above mixture into individual wells of several sterile flat-bottomed microtiter plates. The plates were incubated at 37$^\circ$C in a humidified atmosphere consisting of 6% CO$_2$ and 94% air. Fresh selection medium was added on alternate days for the next 11 days. On day 11, the microculture supernatants were tested by the ELISA assay described above for antibodies reactive with E. coli J5 endotoxin core. Positive cultures were expanded in number and transferred to 35 cm$^2$ flasks. The medium from these cultures was retested, and those maintaining antibody secretion were cryopreserved and stored at -179$^\circ$C in liquid nitrogen vapor phase.

One of the positive hybridoma cultures described above, and one derived from the second fusion experiment, were selected for cloning by limiting dilution. 100 l aliquots containing 0-1 cells were transferred to several hundred individual wells in sterile flat-bottomed microtiter plates that had been previously seeded with 1.0 x 10$^5$ mouse tumor macrophages (P388D1) per well. The macrophages are available from several commercial sources. They serve as "feeder" cells for the hybridomas, and were irradiated (Cobalt 60 source, 1000 Rads) prior to use to prevent multiplication in culture. After 14 days, the cloned cultures were tested again by ELISA assay for antibodies reactive with endotoxin core, and one positive clone from each of the two parental cultures was selected for further study. These cloned hybridomas, designated J5-1 (ATCC HB 8297) and J5-2 (ATCC HB 8298) were expanded in numbers, cryopreserved, and stored in the same manner as the parental cell lines from which they were derived.

Two methods were employed to demonstrate that the production of useful quantities of monoclonal antibodies from the J5-1 and J5-2 hybridomas was possible. An in vitro method utilized stationary cultures of both cell lines grown in 75 cm$^2$ culture flasks containing RPMI 1640 medium supplemented as described above. It was convenient, after 5-7 days of incubation at 37$^\circ$C under 6% CO$_2$, to obtain 1-2 liters of culture fluid containing approximately 10 $\mu$g/ml of J5-1 antibody and 1 $\mu$g/ml of the J5-2 antibody.

A second, in vivo method for obtaining large amounts of monoclonal antibodies involved the adaptation of the J5-1 and J5-2 cell lines to growth as "ascites" tumors. Female BALB/c mice were "primed" by intraperitoneal injection of 0.5 ml of pristane (2,6,10,14-tetramethylpentadecane). Pristane is a sterile irritant which elicits a serous secretion ("ascites") in the peritoneal cavity of mice which acts as a growth medium. Approximately 7-10 days following the pristane injection, aliquots containing 1.0 x 10$^6$ actively growing hybridoma cells harvested from in vitro cultures (described above) were inoculated into the peritoneal cavities of primed mice. Hybridoma cells were serially passaged at 1-2 week intervals to freshly primed mice. After 3-4 such passages, the J5-1 and J5-2 hybridomas became well-adapted ascites tumors, growing rapidly in the fluid microenvironment of the mouse peritoneal cavity and secreting large quantities (e.g., 2-10 mg/ml) of monoclonal antibodies reactive with endotoxin core. Routinely, 20-30 ml of ascites fluid was removed from each mouse by aspiration. The antibody-secreting tumor cells were separated from the antibody-containing fluid phase and reinjected into other primed mice, and the process was repeated.

A third method that can be employed for large scale production of monoclonal antibodies reactive with endotoxin core is made possible by, and requires, the prior preparation of the hybridomas and monoclonal antibodies of this invention. According to this method, genes that code for monoclonal antibodies reactive with endotoxin core are transferred from the hybridomas which produce them to more rapidly reproducing microorganisms such as bacteria or yeast, and the antibody product of these genes recovered from the microorganism itself or the medium in which the microorganism is grown. This method is based on recombinant DNA technology as originally described by Cohen et al in the Proceedings of the National Academy of Sciences, USA, of the National Academy of Sciences, USA, 70:3240, 1973, (see also U.S. Patent No. 4,237,224). The techniques used to accomplish this method have been described by Ullrich et al, Science, 196:1313, 1977, and Goeddel et al, Proceedings of the National Academy of Sciences, USA, 76:106, 1979, who cloned the genes for rat insulin and human growth hormone, respectively, into bacteria, and by Hitzeman et al, Nature (London), 293:717, 1981, who transferred the human $\alpha$-1 interferon gene into yeast.

The clones designated J5-1 (ATCC HB 8297) and J5-2 (ATCC HB 8298) were derived from two distinct hybridoma cell lines and were prepared as described above. Both clones are stable in tissue culture; J5-1 (ATCC HB 8297) produces approximately 10 $\mu$g/ml of antibody protein and J5-2 (ATCC HB 8298) secretes about 1 $\mu$g/ml. Both clones have also been adapted as mouse ascites tumors, and produce 2-10 mg/ml of antibody in this system.

The immunoglobulins (antibodies) produced by the J5-1 and J5-2 hybridomas are both of the IgGl isotype and subclass, as demonstrated by double gel immunodiffusion (Ouchterlony) analysis employing immunopurified anti-immunoglobulin class - and subclass-specific reagents. The monoclonality of the J5-1 and J5-2 antibodies, previously insured by re-cloning the hybridomas which produced them, was confirmed by SDS-polyacrylamide gel electrophoresis and autoradiography employing biosynthetically labeled antibody samples.

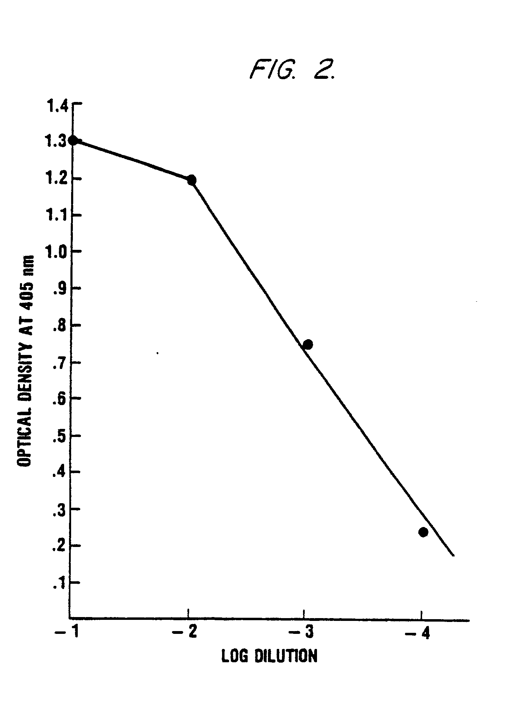

The binding of J5-1 and J5-2 antibodies by endotoxin core was examined using the enzyme-linked immunosorbent assay (ELISA) described above. Purified E. coli J5 core antigen was used to coat the wells of microtiter plates in which the assays were performed and the resulting color reactions measured. A representative "binding curve," shown in Fig. 2, was obtained when dilutions of ammonium sulfate precipitated and redissolved protein from a J5-1 hybridoma culture supernatant were assayed by ELISA. A similar binding curve, but with a slightly different slope, was obtained upon assay of J5-2 culture supernatant.

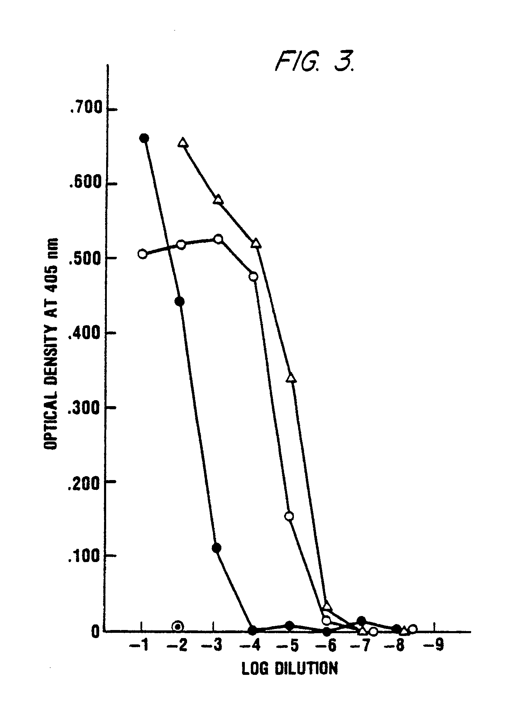

Shown in Fig. 3 are binding curves obtained by ELISA assay of ascites fluid from a mouse which had been intraperitoneally injected with J5-1 antibody-secreting ascites tumor. The parallel binding curves in this figure were obtained by assaying serial dilutions of untreated ascites fluid (O), ammonium sulfate-precipitated and redissolved protein from the same ascites fluid ( △ ), and affinity purified J5-1 monoclonal antibodies ( • ) prepared by passing the ascites fluid over a Sepharose 4B column to which purified E. coli J5 endotoxin had been previously bound. The specificity of J5-1 antibodies produced in ascites fluid was indicated by the results of this immunoabsorption procedure, and by the observed removal of antibody activity from material absorbed with purified endotoxin core ( • in Fig. 3). Antibody activity was detected by ELISA in approximately 1000 times higher dilutions of J5-1 ascites fluid compared with J5-1 culture supernatants, reflecting the higher antibody concentrations obtained by the in vivo production method. Similar amounts of antibody and similar binding curves were obtained in the case of ascites fluid produced by the J5-2 hybridoma.

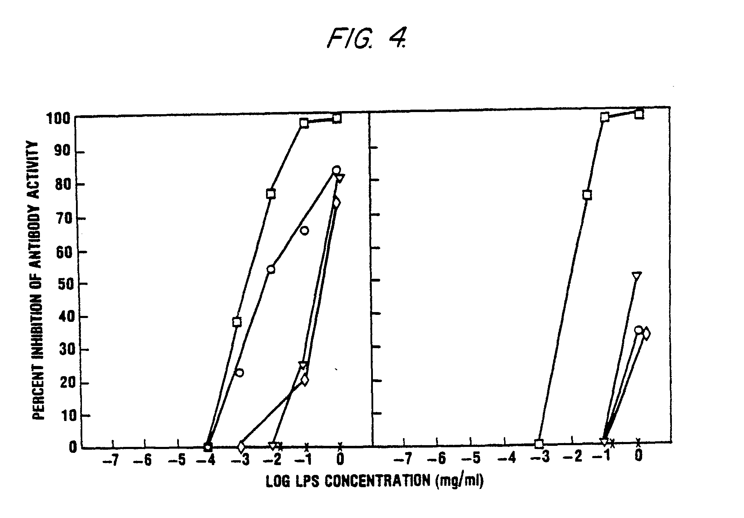

Two salient features of monoclonal antibodies reactive with endotoxin core are their specificity for, as well as their cross-reactivity with, any part of the endotoxin core structures of different Gram-negative bacteria. These characteristics were confirmed by a competitive inhibition ELISA assay of antibodies produced by both the J5-1 (left panel in Fig. 4) and J5-2 (right panel in Fig. 4) hybridomas. Preincubation of both antibodies with purified E. coli J5 endotoxin core ( □ ) abolished their reactivity in the ELISA assay in a dose-dependent manner. This confirmed the specificity of both antibodies for endotoxin core. In addition, it was shown (Fig. 4) that preincubation of J5-1 and J5-2 antibodies with other, heterologous endotoxins, including those obtained from both "rough" and "smooth" organisms and representing different species and genera of bacteria (such as Escherichia coli K235 ( ▽ ), Pseudomonas aeruginosa Fisher Type 2 ( ○ ), and the Salmonella minnesota R595 Re mutant ( ◊ ), also inhibited the reactivity of both antibodies in the ELISA assay. These data, confirmed by double gel immunodiffusion analysis, establish the cross-reactivity of both monoclonal antibodies with endotoxins from a variety of sources.

The functional activity of the J5-1 and J5-2 monoclonal antibodies was evaluated in an in vitro assay which measures the ability of endotoxin to stimulate the proliferation and differentiation of B lymphocytes. This mitogenic activity is shared by most endotoxins and is mediated by the lipid A moiety of the endotoxin core. lipid A also mediates most other biological activities of endotoxins. It is convenient to quantify mitogenic activity in a highly sensitive and widely used in vitro assay system which employs mouse spleen cells.

We used such an in vitro assay to evaluate the ability of J5-1 and J5-2 monoclonal antibodies to neutralize the mitogenic activity of endotoxins from E. coli (J5) and other Gram-negative bacteria. Preincubation of homologous and heterologous endotoxins with J5-1 antibodies caused an inhibition of subsequent mitogenic activity manifested by a decrease in $^3$H-thymidine uptake by mouse spleen cells exposed to the endotoxins in tissue culture for 48 h, as shown in the following table.

Table 1. Neutralization of Lipopolysaccharide-Induced B Cell
Mitogenesis by Monoclonal Antibody Produced by the J5-1 Hybridoma

| Lipopolysaccharide Source | CPM + SEM | |
|---|---|---|
| | Without J5-1 Antibody | With J5-1 Antibody |
| E. coli 0111:B4 (J5 mutant) | 19,456 ± 1,699 | 3,084 ± 301 |
| E. coli K235 | 16,782 ± 481 | 2,755 ± 383 |
| P. aeruginosa (Fisher Type 2) | 25,412 ± 1,034 | 1,848 ± 431 |
| Media control | 1,685 ± 162 | 1,828 ± 290 |

The specificity of the above inhibition was established by the observation that endotoxin-induced B cell mitogenesis was not affected by monoclonal antibodies of the IgGl subclass directed toward antigens other than endotoxin core, and by the fact that neutralization of endotoxin-induced mitogenesis by J5-1 antibodies was demonstrable with endotoxin-responsive B lymphocytes from C3H/Fej mice but not with endotoxin-unresponsive B lymphocytes from C3H/Hej mice.

The data presented in Table 1 thus establish the fact that monoclonal antibodies produced by the J5-1 hybridoma neutralize a biological activity of endotoxin. These data also confirm the cross-reactivity of J5-1 antibodies with endotoxins derived from different genera and species of Gram-negative bacteria. Similar data were obtained with antibodies produced by the J5-2 clone which also specifically inhibited the mitogenic activity of homologous and heterologous endotoxins.

Although the antibodies secreted by the J5-1 and J5-2 hybridomas were similar in their activities, they produced binding curves with distinctive slopes and their endotoxin-neutralising capacities were quantitatively different, suggesting that the two monoclonal antibodies are directed toward two separate sites (epitopes) on the endotoxin core structure. This, in turn, indicates the existence of multiple distinct B lymphocyte clones producing monoclonal antibodies directed at different epitopes, having different binding affinities, and perhaps different functional activities, yet all having in common their reactivity with endotoxin core. The J5-1 and J5-2 antibodies thus represent but two examples of this larger "set" of antibodies defined by their reactivity with any part of endotoxin core. The present invention embraces the disclosed antibodies and the self-reproducing carrier cells ATCC HB8297 and ATCC HB8298 and producing the antibodies.

We next evaluated the cross-protective activity of J5-1 monoclonal antibodies against an actual Gram-negative bacterial infection caused by an organism genetically distinct from the E. coli J5 strain. Monoclonal antibody obtained by ammonium sulfate precipitation of the culture supernatant from an in vitro culture of the J5-1 hybridoma was injected intravenously into 20g Swiss-Webster mice at a dose of 20 $\mu$g/mouse. Control animals received bovine serum albumin (BSA) in the same dose. Eighteen hours later, the mice underwent a 10 sec, 6.25 cm$^2$ flame burn followed by subcutaneous inoculation at the burn site of 6, 10-fold dilutions, 5 mice per dilution, of washed, log phase Pseudomonas aeruginosa (Fisher Type 1) bacteria. Deaths were recorded for three days and median lethal dose (LD$_{50}$) of bacteria calculated by the method of Spearman-Karber (D.J. Finney, Statistical Method in Biological Assay, Third Edition, Macmillan, New York, 1978, pp. 394-401) for the J5-1 and control groups, respectively. The resulting data are shown below.

Table 2. Protective Activity of Monoclonal Antibody Produced by the J5-1 Hybridoma Against Pseudomonas aeruginosa Burn Infections in Mice

| Treatment | Inoculum Size (CFU) | | | | | | LD$_{50}$ |
|---|---|---|---|---|---|---|---|
| | $10^8$ | $10^7$ | $10^6$ | $10^5$ | $10^4$ | $10^3$ | |
| BSA (control) | 0* | 0 | 0 | 80 | 80 | 100 | $1.3 \times 10^5$ |
| Anti-J5 | 0* | 0 | 60 | 100 | 100 | 100 | $1.4 \times 10^6$ |

*Percent survival, five mice per group.

Protection was observed in mice that received J5-1 monoclonal antibody, as indicated by a greater than 10-fold increase in the size of the bacterial innoculum required to kill 50% of the animals in this group. These and similar studies demonstrate the in vivo effectiveness of monoclonal antibodies to endotoxin core in preventing death due to a Gram-negative bacterial infection. In this case, monoclonal antibody to the endotoxin core of the E. coli J5 strain exhibited protective activity against a lethal infection caused by a Pseudomonas aeruginosa organism, thus demonstrating the cross-protective efficacy of this antibody against heterologous Gram-negative bacteria.

In summary, monoclonal antibodies reactive with endotoxin core were prepared by fusing mouse B lymphocytes with plasmacytoma cells and selecting for hybridomas which secreted these specific antibodies. Two distinct monoclonal antibodies reactive with endotoxin core, derived from different hybridoma clones resulting from two separate fusions, were mass-produced in tissue culture and in mouse ascites, thus illustrating the feasibility of preparing these antibodies in large quantities by in vitro and in vivo techniques. The two monoclonal antibodies thus produced, which we designated J5-1 and J5-2, were characterized as described above. Their specific reactivity with homologous and heterologous endotoxins was demonstrated in a binding assay; their ability to neutralize a lipid A-mediated biological activity of endotoxins derived from different Gram-negative bacteria was indicated by the results of mitogenesis assays; and their cross-protective activity against Gram-negative bacterial infections was suggested by passive protection studies carried out in a murine Pseudomonas burn-wound sepsis model.

The novel feature of this invention derives from the selection and reproduction of genes specifying the production of monoclonal antibodies secreted by cell lines ATCC HB 8297 and ATCC HB 8298 reactive with at least part of endotoxin core. In addition, the invention is directed to the actual production of these antibodies

The scope of the invention includes, in addition to the monoclonal antibodies themselves and methods of preparation, in vitro and in vivo processes for producing the antibodies, immuno-diagnostic methods and compositions employing the antibodies, compositions employing the antibodies, research methods and compositions employing the antibodies, purification methods and compositions employing the antibodies, and other methods and compositions employing the antibodies, such methods and compositions predicated upon the specific interaction between the antibodies and endotoxin core.

For the purpose of illustrating the invention, the preceding examples were primarily in terms of hybridomas, and in the case of the examples actually illustrated, these hybridomas resulted from the fusion of mouse B lymphocytes and mouse plasmacytoma cells. However, the essential and novel feature of the present invention is the employment of genes coding for the production of specific antibodies reactive with at least part of endotoxin core and the preparation, employing these genes, of monoclonal antibodies reactive with at least part of endotoxin core. As indicated, the genes employed and the carrier cells employed may originate from any animal species, including man, as long as they result in the production of monoclonal antibodies reactive with at least part of endotoxin core. Likewise, any in vitro or in vivo methods and compositions may be employed for the large-scale production of these antibodies as long as monoclonal antibodies reactive with at least part of endotoxin core result from their use.

When carrier cells ATCC HB 8297 or ATCC HB 8298 are employed in the invention, they are principally characterized by being self-reproducible, and by having genes that code for the production of monoclonal anti-bodies reactive with at least part of endotoxin core. As illustrated in connection with other monoclonal antibodies, these carrier cells can be cell lines such as human-human (Hunter et al., Lancet, 2:798, 1982), human-nonhuman (Nowinski et al., Science, 210:537, 1980), or wholly nonhuman hybridomas (Kohler and Milstein, Nature, 265:495, 1975) or transformed parental lymphoid cells (Steinitz et al., Nature, 269:420, 1977). These references, in combination with the detailed description of the patent, would enable a person skilled in the art to prepare carrier cells of a human or nonhuman animal species containing genes of human or nonhuman origin that code for the production of monoclonal antibodies reactive with endotoxin core having the characteristics of ATCC HB 8297 or ATCC HB 8298. For example, human-human hybridomas producing antibodies reactive with endotoxin core can be prepared in a manner similar to that described above for mouse hybridomas except that spleen cells or peripheral blood lymphocytes obtained from human donors immunized with or previously exposed to endotoxin core and a human myeloma fusion partner such as the HFB-1 cell line (Hunter et al., Lancet, 2:798, 1982) are substituted for mouse spleen and plasmacytoma cells, respectively. Similarly, spleen cells or peripheral blood lymphocytes obtained from human donors immunized with or previously exposed to endotoxin core can be fused with a mouse myeloma fusion partner such as the P3-x63-Ag8.653 cell line (described above in the preparation of a mouse-mouse hybridoma), yielding a self-reproducing human-mouse hybridoma which produces human monoclonal antibody reactive with endotoxin core.

Another approach to the preparation of self-reproducing carrier cells that secrete human or nonhuman monoclonal antibodies reactive with endotoxin core involves virus transformation of the appropriate B lymphocyte clone. Steinitz et al. (Nature, 269:420, 1977) employed such a procedure to prepare specific human antibody to the synthetic hapten NNP (4-hydroxy-3, 5-dinitrophenacetic acid). According to this technique, for example, peripheral blood lymphocytes from human donors immunized with or previously exposed to purified endotoxin core can be isolated on Ficoll-Hypaque. A B lymphocyte population enriched in respect to the production of antibodies reactive with endotoxin core is prepared by a method such as rosetting on endotoxin core-coated erythrocytes. Rosetted cells are separated from non-rosetted cells by centrifugation on Ficoll-Hypaque, placed in tissue culture medium, and infected with Epstein-Barr Virus (EBV) obtained, for example, from supernatants from mycoplasma-free B95-8 cell cultures. The EBV-infected B lymphocytes are transformed into continuously proliferating cell lines ("immortalized"), and those secreting antibodies reactive with endotoxin core are identified by ELISA or other appropriate assay and cloned, essentially as described above for hybridomas. The permanent cell lines thus obtained, which produce human monoclonal antibodies reactive with endotoxin core, can be grown, and antibody production maintained indefinitely, in RPMI 1640 plus 20% fetal calf serum or comparable medium.

The procedures outlined above for obtaining human or nonhuman monoclonal antibodies reactive with endotoxin core employing B lymphocytes fused with tumor cells (hybridomas) and virus-transformed B lymphocytes are similar in all respects except the method by which "immortalization" of the appropriate B lymphocyte clone is achieved. Both techniques entail preparation of purified endotoxin core, immunization of a B lymphocyte donor, selection and cloning of self-reproducing carrier cells containing genes specifying the production of monoclonal antibodies reactive with endotoxin core, growth of these cells in continuous culture, and recovery of the monoclonal antibodies produced.

Yet another approach useful for the preparation of self-reproducing carrier cells containing genes specifying the production of monoclonal antibodies reactive with endotoxin core was described earlier in the specification in connection with the large-scale production of these antibodies. This method, which involves the cloning of genes coding for the production of antibodies reactive with endotoxin core into rapidly dividing microorganisms employing recombinant DNA techniques, requires the prior existence of self-reproducing carrier cells (e.g. hybridomas) that contain these genes and that produce the monoclonal antibodies of this invention.

Just as a variety of different systems and methods might be employed to select for and reproduce genes specifying the production of monoclonal antibodies reactive with endotoxin core, so might a variety of antibodies result from these measures that are distinct from the two antibodies illustrated above yet still clearly within the definition of this invention. Once again, the salient feature of such antibodies, for the purposes of this invention, besides their monoclonality, is their reactivity with at least part of endotoxin core. Thus, the invention includes any monoclonal antibody that reacts with at least part of endoxotin core and is producible by hybridoma ATCC HB 8297 or HB 8298.

As previously discussed, some of the major advantages of monoclonal over polyclonal antibodies reactive with endotoxin core derive from their exquisite specificity and the virtually limitless quantities in which they can be produced. These features of monoclonal antibodies to endotoxin core, together with their demonstrated cross-reactivity with endotoxins from diverse Gram-negative bacteria, their ability to neutralize the biological activity of endotoxins, and their protective activity upon administration to animals with Gram-negative infections, suggest a number of important applications and convenient forms in which the antibodies might be used. These applications fall into four major areas, as outlined below.

The monoclonal antibodies of this invention are reagents that may be used to identify endotoxin, or microorganisms bearing endotoxin, in the tissues or body fluids of patients (or animals) infected with these microorganisms, thus permitting rapid and accurate immuno-logical diagnosis of such infections. This form of diagnosis is made possible, in part, by the great specificity of the monoclonal antibodies of this invention compared with conventional, polyclonal antibodies reactive with endotoxin core. A further advantage of immuno-logical diagnosis utilizing monoclonal antibodies is the speed with which this form of diagnosis can be carried out (e.g., hours) compared with diagnosis based on standard microbiological or cultural methods (e.g., days). A still further advantage of immuno-logical diagnosis employing monoclonal antibodies is that prior antibiotic treatment of an infected patient does not necessarily interfere with or prevent diagnosis as it may in the case of standard microbiological diagnostic procedures.

Monoclonal antibodies reactive with endotoxin core are also useful for the immuno-logical detection of endotoxin or endotoxin-bearing microorganisms present as contaminants in water, biologicals, pharmaceuticals or other materials. Detection is convenient, rapid, sensitive, and highly specific. Furthermore, immuno-logical detection of endotoxin employing monoclonal antibodies to endotoxin core is far more specific than the currently employed limulus lysate assay for endotoxin or assays which employ polyclonal antibodies.

A diagnostic composition, or composition useful for detection of endotoxin, in accordance with the present invention, contains a concentration of the antibody effective to diagnose an infection, detect endotoxin, or demonstrate endotoxin-bearing microorganisms. The antibody can be packaged and sold in freeze-dried or other acceptable form for diagnostic use. It may be mixed with a suitable carrier, attached to an appropriate solid phase (e.g., latex particle, protein A-bearing Staphylococcus aureus, or plastic microtiter plate), conjugated with an enzyme or dye, or radiolabeled, depending on what immunological method is employed.

In a diagnostic or detection method in accordance with this invention, the antibodies of the present invention may be mixed with a sample of body fluid or blood or tissue removed from a person (or animal) infected with an endotoxin-bearing microorganism, or sample of water, biological, pharmaceutical or other material contaminated with endotoxin or an endotoxin-bearing microorganism, and the degree of reaction in the resulting mixture measured. The amount of antibody required to carry out the diagnosis or accomplish the detection depends upon factors that include the amount of sample to be tested, the amount of endotoxin or number of microorganisms present, and the type of assay used. The monoclonal antibodies of the present invention can be employed for diagnosis or detection, as described above, in virtually any immunological assay systems, of which immunofluorescence assays, radioimmunoassays, and enzyme-linked immunosorbent assays are examples only. Furthermore, the monoclonal antibodies of this invention can be used in a competitive binding or inhibition assay to measure other antibodies, either monoclonal or polyclonal, reactive with endotoxin core. Consequently, any assay system which employs monoclonal antibodies reactive with endotoxin core is embraced by this invention.

Monoclonal antibodies of this invention are reagents that may be used for the preparation of compositions useful for the immunoprophylaxis or therapy of Gram-negative infections, or their consequences, including septic shock. These clinical applications of the monoclonal antibodies of the invention are supported by their specificity for endotoxin core, their cross-reactivity with most Gram-negative bacteria, their ability to neutralize the biological activity of endotoxin, their cross-protective efficacy in experimental Gram-negative infections, and their producability in virtually limitless supply, thus overcoming a major shortcoming of polyclonal antibodies.

A composition in accordance with the present invention contains a concentration of an antibody reactive with at least part of the endotoxin core of Gram-negative bacteria effective in preventing or treating (i.e., ameliorating) infections caused by endotoxin bearing microorganism, or the consequences of such infections, including septic shock. The antibodies can be packaged and sold in freeze-dried or other acceptable form, and/or mixed with a therapeutically acceptable carrier, such as a balanced aqueous salt solution.

An immunoprophylactic or therapeutic method using the composition according to this invention entails the administration of the monoclonal antibodies of the invention by injection or infusion prior to (prophylaxis) or following (therapy) the onset of an infection caused by a pathogenic, endotoxin-bearing microorganism. The amount of antibody required to prevent or treat such an infection or its consequences depends upon

such factors as the type and severity of the infection, the size and weight of the infected patient, and the effectiveness of other concomitantly employed modes of prophylaxis or therapy.

The monoclonal antibodies of this invention are useful reagents for research related to the structure and function of bacterial endotoxins. Their exquisite specificity allows them to be used for immunochemical and structure-activity analyses of endotoxin, and makes them more useful in these applications than less specific, conventional polyclonal antibodies. Moreover, the demonstrated cross-reactivity of the monoclonal antibodies of this invention with endotoxins from diverse bacteria confers upon them great versatility as investigational reagents.

A composition in accordance with the present invention useful as an investigational reagent contains an amount of antibody effective in providing information upon mixture with endotoxin and subsequent analysis. Determination of the amount of antibody necessary to accomplish a particular research goal depends upon the specific type of investigation involved, and is readily within the skill of one carrying out such research.

Likewise, a research method in accordance with this invention involves mixing the antibodies of the invention with endotoxin in such a way as to identify specific immunodeterminants and their biological or biochemical properties. The concentration of antibody used and the exact experimental format of such investigations depends on the type of research involved and can be readily determined by one skilled in doing such research.

The monoclonal antibodies of this invention are reagents that can be used for the isolation and purification of endotoxin contained in complex mixtures, and for the neutralization and/or removal of endotoxine from solutions which contain them. The novel attributes of monoclonal antibodies reactive with endotoxin core which make them particularly useful for these applications are their great specificity, compared with polyclonal antibodies, and their availability in virtually limitless quantities, which permits their use on a large, industrial or commercial scale.

A composition in accordance with the invention useful in purifying or removing endotoxin from complex mixtures contains an amount of antibody, contained in an appropriate solution or coupled to an appropriate matrix, to permit specific binding of endotoxin.

An immunological method in accordance with this invention effective in purifying or removing endotoxin from complex mixtures entails coupling antibodies of the invention to a suitable matrix (such as cyanogen bromide-activated Sepharose 4B, available from Pharmacia Fine Chemicals, Piscataway, NJ). A complex mixture containing endotoxin can then be passed over a chromatographic column consisting of the monoclonal antibody coupled to the matrix. As a consequence of this procedure, endotoxin contained in the original mixture, or present as a contaminant in a solution, is specifically bound to the monoclonal antibody, which, in turn, is immobilized on the solid matrix. Everything contained in the complex mixture or contaminated solution, except the endotoxin itself, may be readily removed from the solid matrix by washing, and thus separated from the endotoxin which remains tightly bound. Finally, if one wishes to recover the bound endotoxin, now isolated on the solid matrix, various physical-chemical procedures (e.g., utilizing low pH, high ionic strength, or chaotropic ions such as thiocyanate) may be employed to release the endotoxin from the anti-endotoxin antibody. Endotoxin thus released will have been effectively separated from other components of the complex mixture in which it was originally contained, and it will now be highly purified as a consequence of this "affinity purification" or "immunoabsorption" procedure, utilizing the monoclonal antibodies of the invention.

**Claims**

1. A monoclonal antibody of an animal species reactive with at least part of the endotoxin core of Gram-negative bacteria, wherein the antibody is producible by a self-reproducing carrier cell containing antibody coding genes specifying the production of said antibody, characterized in that said carrier cell is the hybridoma ATCC HB 8297.

2. A monoclonal antibody of an animal species reactive with at least part of the endotoxin core of Gram-negative bacteria, wherein the antibody is producible by a self-reproducing carrier cell containing antibody coding genes specifying the production of said antibody, characterized in that said carrier cell is the hybridoma ATCC HB 8298.

3. The monoclonal antibody of claim 1 or claim 2, wherein said antibody is freeze dried.

4. A self-reproducing carrier cell said carrier cell containing genes that code for the production of a monoclonal antibody which is reactive with at least part of the endotoxin core of Gram-negative

EP 0 151 128 B1

bacteria, characterized in that said carrier cell is the hybridoma ATCC HB 8297.

5. A self-reproducing carrier cell said carrier cell containing genes that code for the production of a monoclonal antibody which is reactive with at least part of the endotoxin core of Gram-negative bacteria, characterized in that said carrier cell is a hybridoma ATCC HB 8298.

6. An in vitro process of preparing the monoclonal antibody of claim 1 or claim 2 comprising the steps of:

culturing a self-reproducing carrier cell containing genes that code for a monoclonal antibody reactive with at least part of the endotoxin core of Gram-negative bacteria, and recovering the antibody from said medium.

7. In an immunologic or immunodiagnostic method the improvement comprising using the antibody of claim 1 or claim 2 for detection of endotoxin, endotoxin-bearing microorganisms, or antibodies reactive with endotoxin.

8. An immunologic or immunodiagnostic composition for the detection of endotoxin, endotoxin-bearing microorganisms, or antibodies reactive with endotoxin comprising at least in part the antibody of claim 1 or claim 2.

9. In a method for biochemical, immunological, functional, or other investigational in vitro analyses of endotoxin, the improvement comprising employing, in admixture with an acceptable carrier, the monoclonal antibody of claim 1 or claim 2 effective to result in such analyses.

10. A composition useful for biochemical, immunological, functional or other investigational analyses of endotoxin, comprising, in admixture with an acceptable carrier, the monoclonal antibody of claim 1 or claim 2 in concentrations effective to result in such analyses.

11. A method for purification of endotoxin comprising contacting in vitro endotoxin to be purified with the monoclonal antibody of claim 1 or claim 2 in admixture with an acceptable carrier or coupled to an appropriate matrix, effective to result in such purification.

12. A composition useful for purification of endotoxin comprising, in admixture with an acceptable carrier or coupled to an appropriate matrix, the monoclonal antibody of claim 1 or claim 2 effective to result in such purification.

13. A method for the removal of endotoxin from fluids, solutions, or suspensions in vitro comprising contacting a material from which endotoxin is to be removed with the monoclonal antibody of claim 1 or claim 2, in admixture with a suitable carrier or coupled to an appropriate matrix, effective to result in such removal.

14. A composition useful for the removal of endotoxin from fluids, solutions, or suspensions, in vitro comprising, in admixture with a suitable carrier or coupled to an appropriate matrix, the monoclonal antibody of claim 1 or claim 2 effective to result in such removal.

15. A composition for treating or preventing clinical manifestations of an infection in a human or animal host caused by an endotoxin-bearing microorganism comprising a monoclonal antibody reactive with at least part of the endotoxin core of Gram-negative bacteria in a pharmaceutically acceptable carrier and in concentrations effective to result in the prevention or amelioration of said clinical manifestations.

**Claims for the following Contracting State: AT**

1. A method for preparing a monoclonal antibody of an animal species reactive with at least part of the endotoxin core of Gram-negative bacteria, wherein the antibody is producible by a self-reproducing carrier cell containing antibody coding genes specifying the production of said antibody, **characterized in that** said carrier cell is the hybridoma ATCC HB 8297.

2. A method for preparing a monoclonal antibody of an animal species reactive with at least part of the

17

endotoxin core of Gram-negative bacteria, wherein the antibody is producible by a self-reproducing carrier cell containing antibody coding genes specifying the production of said antibody, **characterized in that** said carrier cell is the hybridoma ATCC HB 8298.

3. A method for preparing the monoclonal antibody of claim 1 or claim 2, **wherein** said antibody is freeze dried.

4. A method for preparing a self-reproducing carrier cell said carrier cell containing genes that code for the production of a monoclonal antibody which is reactive with at least part of the endotoxin core of Gram-negative bacteria, **characterized in that** said carrier cell is the hybridoma ATCC HB 8297.

5. A method for preparing a self-reproducing carrier cell said carrier cell containing genes that code for the production of a monoclonal antibody which is reactive with at least part of the endotoxin core of Gram-negative bacteria, **characterized in that** said carrier cell is a hybridoma ATCC HB 8298.

6. An in vitro process of preparing the monoclonal antibody of claim 1 or claim 2 comprising the steps of:

culturing a self-reproducing carrier cell containing genes that code for a monoclonal antibody reactive with at least part of the endotoxin core of Gram-negative bacteria, and recovering the antibody from said medium.

7. In an immunologic or immunodiagnostic method the improvement comprising using the antibody of claim 1 or claim 2 for detection of endotoxin, endotoxin-bearing microorganisms, or antibodies reactive with endotoxin.

8. A method for preparing an immunologic or immunodiagnostic composition for the detection of endotoxin, endotoxin-bearing microorganisms, or antibodies reactive with endotoxin comprising at least in part the antibody of claim 1 or claim 2.

9. In a method for biochemical, immunological, functional, or other investigational in vitro analyses of endotoxin, the improvement comprising employing, in admixture with an acceptable carrier, the monoclonal antibody of claim 1 or claim 2 effective to result in such analyses.

10. A method for preparing a composition useful for biochemical, immunological, functional or other investigational analyses of endotoxin, comprising, in admixture with an acceptable carrier, the monoclonal antibody of claim 1 or claim 2 in concentrations effective to result in such analyses.

11. A method for purification of endotoxin comprising contacting in vitro endotoxin to be purified with the monoclonal antibody of claim 1 or claim 2 in admixture with an acceptable carrier or coupled to an appropriate matrix, effective to result in such purification.

12. A method for preparing a composition useful for purification of endotoxin comprising, in admixture with an acceptable carrier or coupled to an appropriate matrix, the monoclonal antibody of claim 1 or claim 2 effective to result in such purification.

13. A method for the removal of endotoxin from fluids, solutions, or suspensions in vitro comprising contacting a material from which endotoxin is to be removed with the monoclonal antibody of claim 1 or claim 2, in admixture with a suitable carrier or coupled to an appropriate matrix, effective to result in such removal.

14. A method for preparing a composition useful for the removal of endotoxin from fluids, solutions, or suspensions, in vitro comprising, in admixture with a suitable carrier or coupled to an appropriate matrix, the monoclonal antibody of claim 1 or claim 2 effective to result in such removal.

15. A method for preparing a composition for treating or preventing clinical manifestations of an infection in a human or animal host caused by an endotoxin-bearing microorganism comprising a monoclonal antibody reactive with at least part of the endotoxin core of Gram-negative bacteria in a pharmaceutically acceptable carrier and in concentrations effective to result in the prevention or amelioration of said

18

clinical manifestations.

**Revendications**

1.  Anticorps monoclonal d'une espèce animale réactif avec au moins une partie du noyau d'endotoxine de bactéries Gram-négatif, dans lequel l'anticorps peut être produit par une cellule porteuse auto-reproductrice contenant des gènes codant pour l'anticorps spécifiant la production dudit anticorps, caractérisé en ce que ladite cellule porteuse est l'hybridome ATCC HB 8297.

2.  Anticorps monoclonal d'une espèce animale réactif avec au moins une partie du noyau d'endotoxine de bactéries Gram-négatif, dans lequel l'anticorps peut être produit par une cellule porteuse auto-reproductrice contenant des gènes codant pour l'anticorps spécifiant la production dudit anticorps, caractérisé en ce que ladite cellule porteuse est l'hybridome ATCC HB 8298.

3.  Anticorps monoclonal selon la revendication 1 ou 2, dans lequel ledit anticorps est lyophilisé.

4.  Cellule porteuse auto-reproductrice, ladite cellule porteuse contenant des gènes qui codent pour la production d'un anticorps monoclonal qui est réactif avec au moins une partie du noyau d'endotoxine de bactéries Gram-négatif, caractérisé en ce que ladite cellule porteuse est l'hybridome ATCC HB 8297.

5.  Cellule porteuse auto-reproductrice, ladite cellule porteuse contenant des gènes qui codent pour la production d'un anticorps monoclonal qui est réactif avec au moins une partie du noyau d'endotoxine de bactéries Gram-négatif, caractérisé en ce que ladite cellule porteuse est l'hybridome ATCC HB 8298.

6.  Procédé in vitro de préparation de l'anticorps monoclonal selon la revendication 1 ou 2, comprenant les étapes qui consistent à :
    cultiver une cellule porteuse auto-reproductrice contenant des gènes qui codent pour un anticorps monoclonal réactif avec au moins une partie du noyau d'endotoxine de bactéries Gram-négatif et
    récupérer l'anticorps dans ledit milieu.

7.  Dans un procédé immunologique ou immunodiagnostique, l'amélioration qui consiste à utiliser l'anticorps de la revendication 1 ou 2 pour détecter l'endotoxine, des microorganismes porteurs d'endotoxine ou des anticorps réactifs avec l'endotoxine.

8.  Composition immunologique ou immunodiagnostique pour la détection de l'endotoxine, de microorganismes porteurs d'endotoxine ou d'anticorps réactifs avec l'endotoxine, comprenant au moins en partie l'anticorps de la revendication 1 ou 2.

9.  Dans un procédé d'analyse biochimique, immunologique, fonctionnelle ou d'autre analyse expérimentale in vitro de l'endotoxine, l'amélioration qui consiste à employer, en mélange avec un véhicule convenable, l'anticorps monoclonal de la revendication 1 ou 2 efficace pour conduire à ces analyses.

10. Composition utile pour des analyses biochimiques, immunologiques, fonctionnelles ou autres analyses expérimentales in vitro de l'endotoxine, comprenant, en mélange avec un véhicule convenable, l'anticorps monoclonal de la revendication 1 ou 2 en concentrations efficaces pour conduire à ces analyses.

11. Procédé de purification de l'endotoxine, comprenant la mise en contact in vitro de l'endotoxine à purifier avec l'anticorps monoclonal de la revendication 1 ou 2, en mélange avec un véhicule acceptable ou couplé à une matrice appropriée, efficace pour conduire à une telle purification.

12. Composition utile pour la purification de l'endotoxine comprenant, en mélange avec un véhicule acceptable ou couplé à une matrice appropriée, l'anticorps monoclonal de la revendication 1 ou 2 efficace pour conduire à une telle purification.

13. Procédé pour éliminer l'endotoxine de fluides, de solutions ou de suspensions in vitro, comprenant la mise en contact d'une matière de laquelle on doit éliminer l'endotoxine avec l'anticorps monoclonal de la revendication 1 ou 2, en mélange avec un véhicule acceptable ou couplé à une matrice appropriée, efficace pour conduire à une telle élimination.

14. Composition utile pour éliminer l'endotoxine de fluides, de solutions ou de suspensions in vitro, comprenant, en mélange avec un véhicule acceptable ou couplé à une matrice appropriée, l'anticorps monoclonal de la revendication 1 ou 2, efficace pour conduire à une telle élimination.

15. Composition de traitement ou de prévention de manifestations cliniques d'une infection chez un hôte humain ou animal causée par un microorganisme porteur d'endotoxine, comprenant un anticorps monoclonal réactif avec au moins une partie du noyau d'endotoxine de bactéries Gram-négatif dans un véhicule pharmaceutiquement acceptable et en concentrations efficaces pour conduire à la prévention ou à l'amélioration desdites manifestations cliniques.

**Revendications pour l'Etat contractant suivant: AT**

1. Procédé de préparation d'un anticorps monoclonal d'une espèce animale réactif avec au moins une partie du noyau d'endotoxine de bactéries Gram-négatif, dans lequel l'anticorps peut être produit par une cellule porteuse auto-reproductrice contenant des gènes codant pour l'anticorps spécifiant la production dudit anticorps, caractérisé en ce que ladite cellule porteuse est l'hybridome ATCC HB 8297.

2. Procédé de préparation d'un anticorps monoclonal d'une espèce animale réactif avec au moins une partie du noyau d'endotoxine de bactéries Gram-négatif, dans lequel l'anticorps peut être produit par une cellule porteuse auto-reproductrice contenant des gènes codant pour l'anticorps spécifiant la production dudit anticorps, caractérisé en ce que ladite cellule porteuse est l'hybridome ATCC HB 8298.

3. Procédé de préparation de l'anticorps monoclonal selon la revendication 1 ou 2, dans lequel ledit anticorps est lyophilisé.

4. Procédé de préparation d'une cellule porteuse auto-reproductrice, ladite cellule porteuse contenant des gènes qui codent pour la production d'un anticorps monoclonal qui est réactif avec au moins une partie du noyau d'endotoxine de bactéries Gram-négatif, caractérisé en ce que ladite cellule porteuse est l'hybridome ATCC HB 8297.

5. Procédé de préparation d'une cellule porteuse auto-reproductrice, ladite cellule porteuse contenant des gènes qui codent pour la production d'un anticorps monoclonal qui est réactif avec au moins une partie du noyau d'endotoxine de bactéries Gram-négatif, caractérisé en ce que ladite cellule porteuse est l'hybridome ATCC HB 8298.

6. Procédé in vitro de préparation de l'anticorps monoclonal selon la revendication 1 ou 2, comprenant les étapes qui consistent à :
   cultiver une cellule porteuse auto-reproductrice contenant des gènes qui codent pour un anticorps monoclonal réactif avec au moins une partie du noyau d'endotoxine de bactéries Gram-négatif et récupérer l'anticorps dans ledit milieu.

7. Dans un procédé immunologique ou immunodiagnostique, l'amélioration qui consiste à utiliser l'anticorps de la revendication 1 ou 2 pour détecter l'endotoxine, des microorganismes porteurs d'endotoxine ou des anticorps réactifs avec l'endotoxine.

8. Procédé de préparation d'une composition immunologique ou immunodiagnostique pour la détection de l'endotoxine, de microorganismes porteurs d'endotoxine ou d'anticorps réactifs avec l'endotoxine, comprenant au moins en partie l'anticorps de la revendication 1 ou 2.

9. Dans un procédé d'analyse biochimique, immunologique, fonctionnelle ou d'autre analyse expérimentale in vitro de l'endotoxine, l'amélioration qui consiste à employer, en mélange avec un véhicule convenable, l'anticorps monoclonal de la revendication 1 ou 2 efficace pour conduire à ces analyses.

10. Procédé de préparation d'une composition utile pour des analyses biochimiques, immunologiques, fonctionnelles ou autres analyses expérimentales in vitro de l'endotoxine, comprenant, en mélange avec un véhicule convenable, l'anticorps moncclonal de la revendication 1 ou 2 en concentrations efficaces pour conduire à ces analyses.

11. Procédé de purification de l'endotoxine, comprenant la mise en contact in vitro de l'endotoxine à purifier avec l'anticorps monoclonal de la revendication 1 ou 2, en mélange avec un véhicule acceptable ou couplé à une matrice appropriée, efficace pour conduire à une telle purification.

12. Procédé de préparation d'une composition utile pour la purification de l'endotoxine comprenant, en mélange avec un véhicule acceptable ou couplé à une matrice appropriée, l'anticorps monoclonal de la revendication 1 ou 2 efficace pour conduire à une telle purification.

13. Procédé pour éliminer l'endotoxine de fluides, de solutions ou de suspensions in vitro, comprenant la mise en contact d'une matière de laquelle on doit éliminer l'endotoxine avec l'anticorps monoclonal de la revendication 1 ou 2, en mélange avec un véhicule acceptable ou couplé à une matrice appropriée, efficace pour conduire à une telle élimination.

14. Procédé pour préparer une composition utile pour éliminer l'endotoxine de fluides, de solutions ou de suspensions in vitro, comprenant, en mélange avec un véhicule acceptable ou couplé à une matrice appropriée, l'anticorps monoclonal de la revendication 1 ou 2, efficace pour conduire à une telle élimination.

15. Procédé de préparation d'une composition de traitement ou de prévention de manifestations cliniques d'une infection chez un hôte humain ou animal causée par un microorganisme porteur d'endotoxine, comprenant un anticorps monoclonal réactif avec au moins une partie du noyau d'endotoxine de bactéries Gram-négatif dans un véhicule pharmaceutiquement acceptable et en concentrations efficaces pour conduire à la prévention ou à l'amélioration desdites manifestations cliniques.

## Ansprüche

1. Monoklonaler Antikörper einer Tierspezies, der zumindest mit einem Teil des Endotoxinkerns Gram-negativer Bakterien reaktionsfähig ist, wobei der Antikörper herstellbar ist durch eine selbst-reproduzierende Trägerzelle, die Antikörper codierende Gene enthält, die die Herstellung des Antikörpers spezifizieren, **dadurch gekennzeichnet**, daß die Trägerzelle das Hybridoma ATCC HB 8297 ist.

2. Monoklonaler Antikörper einer Tierspezies, der zumindest mit einem Teil des Endotoxinkerns Gram-negativer Bakterien reaktionsfähig ist, wobei der Antikörper herstellbar ist durch eine selbst-reproduzierende Trägerzelle, die Antikörper codierende Gene enthält, die die Herstellung des Antikörpers spezifizieren, **dadurch gekennzeichnet**, daß die Trägerzelle das Hybridoma ATCC HB 8298 ist.

3. Monoklonaler Antikörper nach Anspruch 1 oder Anspruch 2, **dadurch gekennzeichnet**, daß der Antikörper gefriergetrocknet ist.

4. Selbst-reproduzierende Trägerzelle, wobei die Trägerzelle Gene enthält, die für die Herstellung eines monoklonalen Antikörpers codieren, der mit mindestens einem Teil des Endotoxinkerns Gram-negativer Bakterien reaktionsfähig ist, **dadurch gekennzeichnet**, daß die Trägerzelle das Hybridoma ATCC HB 8297 ist.

5. Selbst-reproduzierende Trägerzelle, wobei die Trägerzelle Gene enthält, die für die Herstellung eines monoklonalen Antikörpers codieren, der mit mindestens einem Teil des Endotoxinkerns Gram-negativer Bakterien reaktionsfähig ist, **dadurch gekennzeichnet**, daß die Trägerzelle ein Hybridoma ATCC HB 8298 ist.

6. In-Vitro-Verfahren zum Herstellen des monoklonalen Antikörpers nach Anspruch 1 oder Anspruch 2, umfassend die Schritte:

Kultivieren einer selbst-reproduzierenden Trägerzelle, die Gene enthält, die für einen monoklonalen Antikörper codieren, der mit mindestens einem Teil des Endotoxinkerns Gram-negativer Bakterien reaktionsfähig ist, und

Gewinnen des Antikörpers aus dem Medium.

7. In einer immunologischen oder immundiagnostischen Methode die Verbesserung umfassend Verwenden des Antikörpers nach Anspruch 1 oder Anspruch 2 zum Nachweis von Endotoxin, Endotoxin-tragenden Mikroorganismen oder Antikörpern, die mit Endotoxin reaktionsfähig sind.

8. Immunologische oder immundiagnostische Zusammensetzung für den Nachweis von Endotoxin, Endotoxin-tragenden Mikroorganismen oder Antikörpern, die mit Endotoxin reaktionsfähig sind, umfassend mindestens teilweise den Antikörper nach Anspruch 1 oder Anspruch 2.

9. In einer Methode für biochemische, immunologische, funktionelle oder andere In-Vitro-Untersuchungsanalysen von Endotoxin die Verbesserung umfassend Verwenden, in Beimengung mit einem akzeptablen Träger, des monoklonalen Antikörpers nach Anspruch 1 oder Anspruch 2, wirksam um solche Analysen zu erzielen.

10. Zusammensetzung, nützlich für biochemische, immunologische, funktionelle oder andere Untersuchungsanalysen von Endotoxin, umfassend, in Beimengung mit einem akzeptablen Träger, den monoklonalen Antikörper nach Anspruch 1 oder Anspruch 2 in Konzentrationen, die wirksam sind, solche Analysen zu erzielen.

11. Methode zum Reinigen von Endotoxin, umfassend In-Kontakt-Bringen in vitro von zu reinigendem Endotoxin mit dem monoklonalen Antikörper nach Anspruch 1 oder Anspruch 2 in Beimengung mit einem akzeptablen Träger oder gekuppelt an eine geeignete Matrix, wirksam, um solche Reinigung zu erzielen.

12. Zusammensetzung, nützlich zum Reinigen von Endotoxin, umfassend, in Beimengung mit einem akzeptablen Träger oder gekuppelt an eine geeignete Matrix, den monoklonalen Antikörper nach Anspruch 1 oder Anspruch 2, wirksam, um solche Reinigung zu erzielen.

13. Methode zum Entfernen von Endotoxin aus Flüssigkeiten, Lösungen oder Suspensionen in vitro, umfassend In-Kontakt-Bringen eines Materials, aus dem Endotoxin entfernt werden soll, mit dem monoklonalen Antikörper nach Anspruch 1 oder Anspruch 2, in Beimengung mit einem geeigneten Träger oder gekoppelt an eine geeignete Matrix, wirksam, um solch eine Entfernung zu erzielen.

14. Zusammensetzung, nützlich für das Entfernen von Endotoxin aus Flüssigkeiten, Lösungen, oder Suspensionen in vitro, umfassend, in Beimengung mit eines geeigneten Träger oder gekuppelt an eine geeignete Matrix, den monoklonalen Antikörper nach Anspruch 1 oder Anspruch 2, wirksam, um solche Entfernung zu erzielen.

15. Zusammensetzung zum Behandeln oder Verhindern klinischer Manifestationen einer Infektion in einem menschlichen oder tierischen Wirt, die von einem Endotoxin-tragenden Mikroorganismus verursacht ist, umfassend einen monoklonalen Antikörper, der mit mindestens einem Teil des Endotoxinkerns Gram-negativer Bakterien reaktionsfähig ist, in einem pharmazeutisch akzeptablen Träger und in Konzentrationen, wirksam, um die Verhinderung oder Verbesserung der klinischen Manifestationen zu erzielen.

**Patentansprüch für folgenden Vertragsstaat: AT**

1. Verfahren zum Herstellen eines monoklonalen Antikörpers einer Tierspezies, der zumindest mit einem Teil des Endotoxinkerns Gram-negativer Bakterien reaktionsfähig ist, wobei der Antikörper herstellbar ist durch eine selbst-reproduzierende Trägerzelle, die Antikörper codierende Gene enthält, die die Herstellung des Antikörpers spezifizieren, **dadurch gekennzeichnet,** daß die Trägerzelle das Hybridoma ATCC HB 8297 ist.

2. Verfahren zum Herstellen eines monoklonalen Antikörpers einer Tierspezies, der zumindest mit einem Teil des Endotoxinkerns Gram-negativer Bakterien reaktionsfähig ist, wobei der Antikörper herstellbar ist durch eine selbst-reproduzierende Trägerzelle, die Antikörper codierende Gene enthält, die die Herstellung des Antikörpers spezifizieren, **dadurch gekennzeichnet**, daß die Trägerzelle das Hybridoma ATCC HB 8298 ist.

3. Verfahren zum Herstellen des monoklonalen Antikörpers nach Anspruch 1 oder Anspruch 2, **dadurch gekennzeichnet**, daß der Antikörper gefriergetrocknet ist.

4. Verfahren zum Herstellen einer selbst-reproduzierenden Trägerzelle, wobei die Trägerzelle Gene enthält, die für die Herstellung eines monoklonalen Antikörpers codieren, der mit mindestens einem Teil des Endotoxinkerns Gramnegativer Bakterien reaktionsfähig ist, **dadurch gekennzeichnet**, daß die Trägerzelle das Hybridoma ATCC HB 8297 ist.

5. Verfahren zum Herstellen einer selbst-reproduzierenden Trägerzelle, wobei die Trägerzelle Gene enthält, die für die Herstellung eines monoklonalen Antikörpers codieren, der mit mindestens einem Teil des Endotoxinkerns Gramnegativer Bakterien reaktionsfähig ist, **dadurch gekennzeichnet**, daß die Trägerzelle ein Hybridoma ATCC HB 8298 ist.

6. In-vitro-Verfahren zum Herstellen des monoklonalen Antikörpers nach Anspruch 1 oder Anspruch 2, umfassend die Schritte:

Kultivieren einer selbst-reproduzierenden Trägerzelle, die Gene enthält, die für einen monoklonalen Antikörper codieren, der mit mindestens einem Teil des Endotoxinkerns Gram-negativer Bakterien reaktionsfähig ist, und

Gewinnen des Antikörpers aus dem Medium.

7. In einer immunologischen oder immundiagnostischen Methode die Verbesserung umfassend Verwenden des Antikörpers nach Anspruch 1 oder Anspruch 2 zum Nachweis von Endotoxin, Endotoxin-tragenden Mikroorganismen oder Antikörpern, die mit Endotoxin reaktionsfähig sind.

8. Verfahren zum Herstellen einer immunologischen oder immundiagnostischen Zusammensetzung für den Nachweis von Endotoxin, Endotoxin-tragenden Mikroorganismen oder Antikörpern, die mit Endotoxin reaktionsfähig sind, umfassend mindestens teilweise den Antikörper nach Anspruch 1 oder Anspruch 2.

9. In einer Methode für biochemische, immunologische, funktionelle oder andere in-vitro-Untersuchungsanalysen von Endotoxin die Verbesserung umfassend Verwenden, in Beimengung mit einem akzeptablen Träger, des monoklonalen Antikörpers nach Anspruch 1 oder Anspruch 2, wirksam um solche Analysen zu erzielen.

10. Verfahren zum Herstellen einer Zusammensetzung, nützlich für biochemische, immunologische, funktionelle oder andere Untersuchungsanalysen von Endotoxin, umfassend, in Beimengung mit einem akzeptablen Träger, den monoklonalen Antikörper nach Anspruch 1 oder Anspruch 2 in Konzentrationen, die wirksam sind, solche Analysen zu erzielen.

11. Methode zum Reinigen von Endotoxin, umfassend In-Kontakt-Bringen in vitro von zu reinigendem Endotoxin mit dem monoklonalen Antikörper nach Anspruch 1 oder Anspruch 2 in Beimengung mit einem akzeptablen Träger oder gekuppelt an eine geeignete Matrix, wirksam, um solche Reinigung zu erzielen.

12. Verfahren zum Herstellen einer Zusammensetzung, nützlich zum Reinigen von Endotoxin, umfassend, in Beimengung mit einem akzeptablen Träger oder gekuppelt an eine geeignete Matrix, den monoklonalen Antikörper nach Anspruch 1 oder Anspruch 2, wirksam, um solche Reinigung zu erzielen.

13. Methode zum Entfernen von Endotoxin aus Flüssigkeiten, Lösungen oder Suspensionen in vitro, umfassend In-Kontakt-Bringen eines Materials, aus dem Endotoxin entfernt werden soll, mit dem monoklonalen Antikörper nach Anspruch 1 oder Anspruch 2, in Beimengung mit einem geeigneten Träger oder gekoppelt an eine geeignete Matrix, wirksam, um solch eine Entfernung zu erzielen.

14. Verfahren zum Herstellen einer Zusamensetzung, nützlich für das Entfernen von Endotoxin aus Flüssigkeiten, Lösungen oder Suspensionen in vitro, umfassend, in Beimengung mit einem geeigneten Träger oder gekuppelt an eine geeignete Matrix, den monoklonalen Antikörper nach Anspruch 1 oder Anspruch 2, wirksam, um solche Entfernung zu erzielen.

15. Verfahren zum Herstellen einer Zusammensetzung zum Behandeln oder Verhindern klinischer Manifestationen einer Infektion in einem menschlichen oder tierischen Wirt, die von einem Endotoxintragenden Mikroorganismus verursacht ist, umfassend einen monoklonalen Antikörper, der mit mindestens einem Teil des Endotoxinkerns Gram-negativer Bakterien reaktionsfähig ist, in einem pharmazeutisch akzeptablen Träger und in Konzentrationen, wirksam, um die Verhinderung oder Verbesserung der klinischen Manifestationen zu erzielen.

## FIG. 1.

FIG. 2.

FIG. 3.

## FIG. 4.